# EUROPÄISCHE PATENTSCHRIFT

(11) **EP 1 654 324 B1**
(45) Veröffentlichungstag und Bekanntmachung des Hinweises auf die Patenterteilung: **27.12.2006**
(21) Anmeldenummer: 04741269.7
(22) Anmeldetag: 26.07.2004
(51) Int. Cl.: C08L 75/04, C07C 311/03, C07C 311/04, C07C 311/07, C07C 311/08, C07D 263/48, C07D 277/52, C07D 241/20

(54) **SULFONAMID-ANIONEN ALS KATALYSATOREN FÜR DIE NCO-OLIGOMERISIERUNG**
SULPHONAMIDE ANIONS AS CATALYSTS FOR NCO OLIGOMERISATION
ANIONS DE SULFONAMIDE COMME CATALYSEURS D'OLIGOMERISATION NCO

(30) Priorität: 07.08.2003 DE 10336186
(43) Veröffentlichungstag der Anmeldung: 10.05.2006
(73) Patentinhaber: Bayer MaterialScience AG, 51368 Leverkusen (DE)
(72) Erfinder: KÖCHER, Jürgen, 40764 Langenfeld (DE); HALPAAP, Reinhard, 51519 Odenthal (DE)
(86) Internationale Anmeldenummer: PCT/EP2004/008342
(87) Internationale Veröffentlichungsnummer: WO 2005/017037

(56) Entgegenhaltungen:
- EP-A- 0 339 396
- WO-A-86/04911
- US-A- 3 582 501
- US-A- 4 423 197

## Beschreibung

Die vorliegende Erfindung betrifft die Verwendung von Sulfonamid-Salzen als Katalysatoren für die Oligomerisierung von Isocyanaten sowie ein Verfahren zur NCO-Oligomerisierung unter Verwendung der erfindungsgemäßen Katalysatoren.

Da monomere Diisocyanate als Vemetzer in Polyurethan-Beschichtungssystemen aufgrund ihrer Flüchtigkeit und ihrer toxikologischen Eigenschaften nicht eingesetzt werden können, bedient man sich in der Regel der monomerenarmen höhermolekularen Derivate z.B. auf Uretdion- Isocyanurat-, Biuret-, Urethan- oder Allophanatbasis. Eine Übersicht zu diesen Polyisocyanaten und ihrer Herstellungsweise ist exemplarisch in J. Prakt. Chem./Chem. Ztg. 1994, 336, 185-200 gegeben.

Die Oligomerisierung von Isocyanaten durch Reaktion von typischerweise zwei oder drei NCO-Funktionen untereinander führt zu den Strukturen der folgenden Formeln 1-3, wobei die Uretdion- (Typ 1) und die Isocyanurat-Struktur (Typ 2) die technisch wichtigen sind.

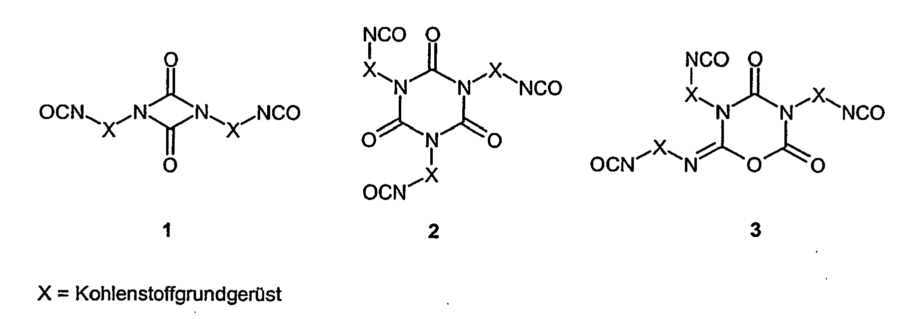

Als Katalysatoren für diese Oligomerisierung ist eine Vielzahl kovalenter und ionischer Katalysatoren in der Literatur beschrieben worden (J. Prakt. Chem./Chem. Ztg. 1994, 336, 185-200). Nicht geladene, kovalent aufgebaute Verbindungen zeigen jedoch gegenüber salzartigen eine deutlich geringere Aktivität, so dass für einen gleichen Umsatz entweder mehr Katalysator eingesetzt werden muss oder eine entsprechend längere Reaktionszeit notwendig ist.

In DE-A 3 100 263, EP-A 339 396 und EP-A 330 966 werden salzartig aufgebaute Katalysatoren wie Carboxylate, Fluoride und Hydroxide für die Isocyanatoligomerisierung beschrieben. Diese zeigen eine hohe Selektivität in Bezug auf Bildung von Isocyanuraten (Typ 2), wobei jedoch kaum oder gar keine Dimerstruktur (Typ 1) gebildet wird.

Es wurde nun gefunden, dass Sulfonamid-Salze ebenfalls hochaktive NCO-Oligomerisierungskatalysatoren sind, wobei Di- und/oder Trimerisierungsprodukte erhalten werden und sich insbesondere bei cycloaliphatischen Isocyanaten das Verhältnis von Di- zu Trimer durch einfache Variation der Substituenten an Schwefel und/oder Stickstoff in weiten Grenzen variieren lässt.

Gegenstand der Erfindung ist daher die Verwendung von Sulfonamid-Salzen der Formel (I) 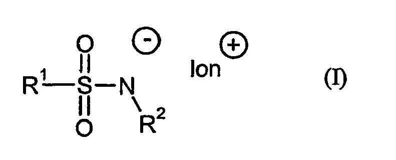 wobei
- R¹, R²: unabhängig voneinander gleiche oder verschiedene gegebenenfalls verzweigte, substituierte und/oder Heteroatom-enthaltende aliphatische, cycloaliphatische, aromatische oder araliphatische Reste sind und
- Ion⁽⁺⁾: ein anorganisches oder organisches Kation ist,
zur Oligomeriserung von Isocyanaten.
Bevorzugt ist
- R¹: ein gegebenenfalls verzweigter und/oder substituierter aliphatischer oder cycloaliphatischer C₁ - C₂₄ Rest, der gegebenenfalls bis zu 3 Heteroatome der Elemente Sauerstoff, Schwefel oder Stickstoff enthält,
- R²: ein Rest der bereits vorstehend allgemein für R² definierten Art und
- Ion⁽⁺⁾: ein Alkali- oder Erdalkalimetallkation oder ein Ammonium- oder Phosphoniumion.

Beispiele für die genannten bevorzugt einzusetzenden Kationen (Ion⁽⁺⁾) sind Li⁺, Na⁺ K⁺, Mg²⁺, Ca²⁺ sowie Ammonium bzw. Phosphonium-Kationen der allgemeinen Formel (II) 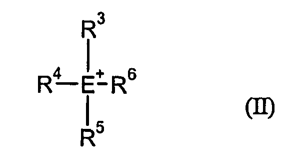 in der
- E: Stickstoff oder Phosphor ist und
- R³, R⁴, R⁵: unabhängig voneinander gleiche oder verschiedene aliphatische, cycloaliphatische oder araliphatische gegebenenfalls Heteroatom-enthaltende Reste oder Wasserstoffatom sind und
- R⁶: der vorstehenden Bedeutung von R³, R⁴, R⁵ oder der Formel (III) entspricht 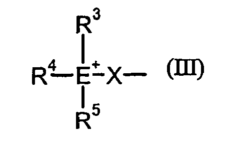
in der
- X: ein zweibindiger, gegebenenfalls Heteroatom-enthaltender aliphatischer, cycloaliphatischer oder araliphatischer C₁ - C₁₂ Rest ist und
- R³, R⁴, R⁵, E: die vorstehend genannte Bedeutung haben.
Besonders bevorzugt ist
- R¹: ein gegebenenfalls verzweigter aliphatischer oder cycloaliphatischer C₁ - C₁₈ Rest, der gegebenenfalls bis zu 3 Heteroatome der Elemente Sauerstoff, Schwefel, Stickstoff und/oder gegebenenfalls Halogen-, Cyanid-, Nitro-, Alkyl-, Aryl-, Alkoxy-, Aryloxy- und/oder Dialkylamino-Substituenten aufweist,
- R²: ein Rest, der der besonders bevorzugten Art von R¹ entspricht oder ein Rest der Gruppe Phenyl-, Tolyl-, Naphthyl-, Biphenyl-, Phenantryl-, Pyrrolidin-, Piperidin-, Piperazin-, Morpholin-, Pyrrol-, Imidazol-, Pyrazol-, Indol-, Indazol-, Pyridin-, Pyrimidin-, Pyridazin-, Pyrazin-, Chinolin-, Isochinolin-, Phthalazin-, Chinoxalin-, Chinazolin-, Thiazol-, Benzothiazol-, Isothiazol-, Oxazol-, Benzoxazol-, Isothiazol-, Benzisoxazol-, Furan-, Benzofuran-, Thiophen-, Benzothiophen ist, der gegebenenfalls ein oder mehrere Substituenten aus der Gruppe Halogen-, Nitro-, Cyanid-, Carboxy-, Carboxyalkyl-, Carboxyaryl-, Alkyl-, Aryl-, Alkoxy-, Aryloxy-, Dialkylamino- aufweist und
- Ion⁽⁺⁾: Li⁺, Na⁺, K⁺ oder ein einwertiges Ammonium- oder Phosphoniumkation der allgemeinen Formel (II) ist, in welcher
E Stickstoff oder Phosphor ist und
- R³, R⁴, R⁵, R⁶: unabhängig voneinander gleiche oder verschiedene aliphatische, cycloaliphatische oder araliphatische gegebenenfalls Heteroatom-enthaltende C₁ - C₁₈ Reste sind.
Ganz besonders bevorzugt ist
- R¹: ein Rest der vorstehend für R¹ besonders bevorzugten Art,
- R²: ein Rest, der R¹ entspricht oder ein Rest der Gruppe Phenyl-, Pyrrolidin-, Piperidin-, Piperazin-, Morpholin-, 2-Pyrimidinyl-, 2-Thiazolyl-, 2-Benzthiazolyl-, 2-Pyrazyl-, 2-Pyridyl- und 4-Pyridyl- und
- Ion(+): ein einwertiges Kation der vorstehend für Ion⁽⁺⁾ besonders bevorzugten Art.

Ein weiterer Gegenstand der Erfindung ist ein Verfahren zur Oligomerisierung von Isocyanaten, bei dem
a) ein oder mehrere organische Verbindungen einer mittleren NCO-Funktionalität ≥ 1 in Anwesenheit
b) eines Katalysators enthaltend ein oder mehrere Sulfonamid-Salze der Formel (I) und
c) optional Lösungsmitteln
oligomerisiert werden.

In das erfindungsgemäße Verfahren können in Komponente a) alle dem Fachmann bekannten aliphatischen, cycloaliphatischen, araliphatischen und/oder aromatischen Isocyanate einer NCO-Funktionalität ≥ 1, bevorzugt ≥ 2 einzeln oder in beliebigen Mischungen untereinander eingesetzt werden, wobei es unerheblich ist ob diese nach Phosgen- oder phosgen-freien Verfahren hergestellt wurden.

Bevorzugt werden aliphatische, cycloaliphatische und/oder araliphatische Isocyanate der vorstehend genannten Art eingesetzt, wobei diese ein Kohlenstoffgrundgerüst (ohne die enthaltenen NCO-Gruppen) von 3 bis 30, bevorzugt 4 bis 20 Kohlenstoffatomen aufweisen.

Besonders bevorzugte Verbindungen der Komponente a) entsprechen der vorstehend genannten Art mit aliphatisch und/oder cycloaliphatisch gebundene NCO-Gruppen wie beispielsweise Bis-(isocyanatoalkyl)ether, Bis- und Tris-(isocyanatoalkyl)-benzole, -toluole, sowie -xylole, Propandiisocyanate, Butandiisocyanate, Pentandiisocyanate, Hexandiisocyanate (z.B. Hexamethylendiisocyanat, HDI), Heptandiisocyanate, Octandiisocyanate, Nonandiisocyanate (z.B. Trimethyl-HDI (TMDI) in der Regel als Gemisch der 2,4,4- und 2,2,4-Isomeren), Nonantri-isocyanate (z.B. 4-Isocyanatomethyl-1,8-octandüsocyanat), Dekandiisocyanate, Dekantriisocyanate, Undekandiisocyanate, Undekantriisocyanate, Dodecandiisocyanate, Dodecantriisocyanate, 1,3- sowie 1,4-Bis-(isocyanatomethyl)cyclohexane (H₆XDI), 3-Isocyanatomethyl-3,5,5-trimethylcyclohexylisocyanat (Isophorondiisocyanat, IPDI), Bis-(4-isocyanatocyclohexyl)methan (H₁₂MDI), Bis-(isocyanatomethyl)norboman (NBDI), 3(4)-Isocyanatomethyl-1-methyl-cyclohexylisocyanat (IMCI).

Ganz besonders bevorzugte Verbindungen der Komponente a) sind Hexamethylendiisocyanat (HDI), Trimethyl-HDI (TMDI), 2-Methylpentan-1,5-diisocyanat (MPDI), Isophorondiisocyanat (IPDI), 1,3- sowie 1,4-Bis(isocyanatomethyl)cyclohexan (H₆XDI), Bis(isocyanatomethyl)norbornan (NBDI), 3(4)-Isocyanatomethyl-1-methyl-cyclohexylisocyanat (IMCI) und/oder 2,4'- bzw. 4,4'-Bis(isocyanatocyclohexyl)methan (H₁₂MDI) oder Gemische dieser Isocyanate.

Die anteilige Verwendung monofunktioneller Isocyanate ist ebenfalls möglich.

Im erfindungsgemäßen Verfahren beträgt die Menge des Katalysators b) 0,01 bis 10 mol-%, bevorzugt 0,05 bis 5 mol-%, besonders bevorzugt 0,1 bis 3 mol-% bezogen auf die Menge der Komponente a), wobei sich hier die mol%-Angaben auf die gesamte Stoffmenge in mol des eingesetzten Isocyanates der Komponente a) beziehen.

Bevorzugt werden als Katalysator b) des erfindungsgemäßen Verfahrens ausschließlich Sulfonamid-Salze der Formel (I) eingesetzt.

Der Katalysator b) kann ungelöst als reine Verbindung oder in Form einer Lösung im erfindungsgemäßen Verfahren eingesetzt werden. Das Lösungsmittel ist dabei so zu wählen, dass es den Katalysator zwar molekular oder ionisch dissoziiert löst, nicht jedoch das bzw. die Sulfonamidanion(en) durch chemische Reaktionen in seiner/ihrer Zusammensetzung und/oder molekularen Struktur dabei verändert wird/werden. Gleichzeitig muss das Lösungsmittel gegenüber NCO-Funktionen entweder inert sein oder darf mit Isocyanaten nur unter Ausbildung von Hamstoff-, Biuret-, Urethan- oder Allophanat-Gruppen reagieren.

Sofern der Katalysator b) als Lösung eingesetzt wird, werden bevorzugt geradkettige oder verzweigte C₁ - C₂₀, bevorzugt C₁ - C₁₀ Alkohole mit einer OH-Funktionalität ≥ 1 wie beispielsweise Methanol, Ethanol, 1- sowie 2-Propanol, die isomeren Butanole, 2-Ethylhexanol, 2-Ethylhexan-1,3-diol, 1,3-sowie 1,4-Butandiol oder 1-Methoxy-2-propanol verwendet.

In einer bevorzugten Ausführungsform der Erfindung wird der Katalysator b) als Lösung eingesetzt.

Im erfindungsgemäßen Verfahren können als Komponente c) gegebenenfalls Lösungsmittel mitverwendet werden, wobei bevorzugt außer dem gegebenenfalls verwendeten Katalysatorlösungsmittel keine weiteren Lösungsmittel als Komponente c) eingesetzt werden.

Das erfindungsgemäße Verfahren wird bevorzugt bei Temperaturen von 0 bis 100°C, besonders bevorzugt 20 bis 100°C durchgeführt.

Selbstverständlich kann falls notwendig das Verfahren auch unter erhöhtem oder vermindertem Druck durchgeführt werden.

Das erfindungsgemäße Verfahren kann sowohl kontinuierlich als auch diskontinuierlich geführt werden. Unter einem kontinuierlichen Verfahren wird z.B. die Herstellung in einem Rohrreaktor oder mit Hilfe von Kesselkaskaden verstanden, während diskontiuierliche Verfahren z.B. Verfahren in einem Kessel (Batch-Verfahren) oder einem Kolben sind.

In einer bevorzugten Ausführungsform der Erfindung wird die NCO-Oligomerisierung bis zu einem Umsatz von 10-60 mol-% bezogen auf die Gesamtmenge der ursprünglich vorhandenen NCO-Gruppen geführt, die Oligomerisierungsreaktion abgebrochen und nicht umgesetztes Isocyanat z.B. durch Destillation gegebenenfalls unter vermindertem Druck abgetrennt, wobei das oligomerisierte Isocyanat als Harz erhalten wird.

Zum Abbruch der Oligomerisierungsreaktion eignen sich prinzipiell alle dem Fachmann bekannten Techniken (J. Prakt. Chem./Chem. Ztg. 1994, 336, 185-190). Dazu gehört die Entfernung des Katalysators z.B. durch Extraktion oder Filtration gegebenenfalls unter Zuhilfenahme eines adsorptiv wirkenden Trägermaterials, die Inaktivierung des Katalysatorsystems durch thermische Behandlung und/oder durch Zugabe von Säuren oder Säurederivaten wie Benzoylchlorid, Phthaloylchlorid, phosphinige-, phosphonige- oder phosphorige Säure, Phosphin-, Phosphon- oder Phosphorsäure oder die sauren Ester der oben genannten Phosphor-enthaltenden Säuren. Bevorzugte Abstopper sind Mono- oder Dialkylphosphate wie (Di)butylphosphat, (Di)octylphosphat oder (Di)trihexylphosphat, Schwefelsäure oder ihre sauren Ester oder Sulfonsäuren, wie bevorzugt Methansulfonsäure und p-Toluolsulfonsäure oder Sulfonsäureester wie beispielsweise p-Toluolsulfonsäuremethylester

Die zur Abstoppung der Reaktion benötigte Menge des Katalysatorgiftes richtet sich dabei nach Menge des aktiven Katalysators. Im Allgemeinen werden 70-150 mol-% Abstopper bezogen auf die ursprünglich eingesetzte Katalysatormenge verwendet, bevorzugt ist der Einsatz äquimolarer Mengen Abstopper bezogen auf die eingesetzte Katalysatormenge.

Die nach dem erfindungsgemäßen Verfahren erhaltenen Polyisocyanate lassen sich durch die üblichen Verfahren des Standes der Technik, wie z.B. Dünnschichtdestillation, Extraktion, Kristallisation und/oder Molekulardestillation isolieren und reinigen. Sie fallen dabei als farblose oder nur schwach gefärbte Flüssigkeiten oder Feststoffe an.

Besonders vorteilhaft an den erfindungsgemäßen Katalysatoren zur Isocyanat-Oligomerisierung ist ihre hohe Selektivität zur Isocyanurat- und gegebenenfalls gleichzeitigen Uretdionbildung, wobei sie hochaktiv sind und nur geringe bis gar keine Iminooxadiazindion-Anteile gebildet werden. Besonders im Fall der cycloaliphatischen Isocyanate zeigen die erfindungsgemäßen Katalysatoren zusätzlich eine für ionische Katalysatoren überraschend hohe Neigung zur Bildung von NCO-Dimeren.

Die erfindungsgemäß hergestellten Polyisocyanate stellen vielseitig verwendbare Ausgangsmaterialien zur Herstellung von Polymeren dar, wie z.B. gegebenenfalls geschäumten Kunststoffen oder Polyurethan-Lacken, insbesondere zur Herstellung von Ein- und ZweikomponentenPolyurethanlacken, Beschichtungsmitteln, Klebstoffen und Zuschlagstoffen für die Anwendung auf Materialien wie z.B. Holz, Kunststoff, Leder, Metall, Papier, Beton, Mauerwerk, Keramik und Textil.

### Beispiele

Die Prozentangaben des Umsatzes berechnen sich durch Division der Menge an umgesetztem Isocyanat durch die Gesamtmenge des eingesetzten Isocyanats multipliziert mit 100. Alle weiteren Prozentangaben sind, soweit nicht anders vermerkt, als Gewichtsprozente zu verstehen.

Die Ermittlung des NCO-Gehaltes der in den Beispielen und Vergleichsbeispielen beschriebenen Harze erfolgte durch Titration gemäß DIN 53 185.

### Verwendete Abkürzungen:

DMSO: Dimethylsufoxid
n-Bu- oder Bu: n-Butyl
i-PrOH: Isopropanol

Die dynamischen Viskositäten der Polyisocyanatharze wurden bei 23°C mit dem Viskosimeter VT 550, Platte-Kegel Messanordnung PK 100, der Fa. Haake (Karlsruhe, Deutschland) bestimmt. Durch Messungen bei unterschiedlichen Schergeschwindigkeiten wurde sichergestellt, dass das Fließverhalten der beschriebenen erfindungsgemäßen Polyisocyanatmischungen wie auch das der Vergleichsprodukte dem idealer Newtonscher Flüssigkeiten entspricht. Die Angabe der Schergeschwindigkeit kann deshalb entfallen.

Zur Bestimmung des Isocyanat-Umsatzes wurden 20 bis 40 mg der hergestellten Reaktionsmischungen in 3 ml Chloroform gelöst und mit Hilfe der Gelpermeationschromatographie (Säule MZ-Gel Sdplus 500A 5 µm, MZ-Analysentechnik, Mainz, DE) untersucht. Auf eine Katalysatordeaktivierung konnte aufgrund der hohen Verdünnung der Messlösung verzichtet werden. Aus der ermittelten Menge an monomerem Isocyanat kann der NCO-Umsatz bzw. die Harzausbeute berechnet werden.

Die anschließende Bestimmung der Selektivität des eingesetzten Katalysators wurde durch Untersuchung der gebildeten Strukturtypen 1 - 3 durchgeführt. Dazu wurden 30 µl des Reaktionsgemischs zwischen KBr-Platten IR-spektroskopisch (Spektrometer: Arid-Zone^{®} der Firma Bomem, Québec, Kanada, Scanzahl 10, Auflösung 2 cm⁻¹) vermessen. Anhand der Schwingungen bei 1760 cm⁻¹ (Strukturtyp 1), 1690 cm⁻¹ (Strukturtyp 2) und 1780 cm⁻¹ (Strukturtyp 3) kann die Bildung der Strukturtypen 1-3 nachgewiesen werden. Für den Fall, dass mehr als nur ein Strukturtyp gebildet wurde, wurden zur quantitativen Auswertung ¹³C-NMR-Messungen durchgeführt und die Produktmengen über die Signalintegration berechnet.

Für die ¹³C-NMR-Analytik wurden 0,5 ml des jeweiligen Reaktionsgemischs mit, bezogen auf die eingesetzten Katalysatormengen, stöchiometrischen Mengen an Di-n-butylphosphat versetzt, um den Katalysator zu deaktivieren und eine Weiterreaktion zu unterbinden. Durch Zugabe von deuteriertem Chloroform wurden etwa eine Konzentration von 50 Gew.-% Harz eingestellt. Die Messungen erfolgten auf einem DPX 400 der Fa. Bruker, Karlsruhe, DE, bei einer ¹³C-ResonanzFrequenz von 100 MHz. Als Referenz für die ppm-Skala wurde Tetramethylsilan als interner Standard verwendet. Daten für die chemische Verschiebung der infrage kommenden Verbindungen sind der Literatur entnommen (vgl. Die Angewandte Makromolekulare Chemie 1986, 141, 173-183 und darin zitierten Literatur) bzw. durch Vermessung von Modellsubstanzen gewonnen worden.

### Herstellung der erfindungsgemäßen Katalysatoren

### Beispiel 1: Herstellung von n-Butyl-N-n-propylsulfonamid

In 85 ml Methylenchlorid wurden 6,9 ml n-Propylamin (4,9 g, 84 mmol) und 11,6 ml Triethylamin (8,5 g, 84 mmol) bei Raumtemperatur gelöst. Zu dieser Lösung wurden ebenfalls bei Raumtemperatur 10,9 ml n-Butansulfonsäurechlorid (13,1 g, 84 mmol) innerhalb von 1 h zugetropft. Nach 20 h Rühren wurde das Reaktionsgemisch zweimal mit 50 ml Wasser gewaschen. Die organische Phase wurde mit Magnesiumsulfat getrocknet, das Methylenchlorid abdestilliert und der verbleibende ölige Rückstand im Vakuum getrocknet. Es wurden 13,5 g der Zielverbindung erhalten, deren Konstitution mittels NMR-Spektroskopie gesichert wurde.

### Beispiel 2: Herstellung von n-Bntyl-N-(2-methoxyethyl)sulfonamid

In 72 ml Methylenchlorid wurden 6,4 ml 2-Methoxyethylamin (5,5 g, 73,7 mmol) und 10,2 ml Triethylamin (7,4 g, 73,7 mmol) bei Raumtemperatur gelöst. Zu dieser Lösung wurden ebenfalls bei Raumtemperatur 9,6 ml n-Butansulfonsäurechlorid (11,5 g, 73,7 mmol) innerhalb von einer Stunde zugetropft. Nach 1 h Rühren bei Raumtemperatur wurde das Reaktionsgemisch zweimal mit 100 ml Wasser gewaschen. Die organische Phase wurde mit Magnesiumsulfat getrocknet, das Methylenchlorid abdestilliert und der verbleibende ölige Rückstand im Vakuum getrocknet. Es wurden 12,4 g der Zielverbindung erhalten, deren Konstitution mittels NMR-Spektroskopie gesichert wurde.

### Beispiel 3: Herstellung von n-Butyl-N-4-methylpiperazinyl-sulfonamid

In 72 ml Methylenchlorid wurden 8,9 ml 1-Amino-4-methylpiperazin (8,5 g, 73,7 mmol) und 10,2 ml Triethylamin (7,4 g, 73,7 mmol) bei Raumtemperatur gelöst. Zu dieser Lösung wurden ebenfalls bei Raumtemperatur 9,6 ml n-Butansulfonsäurechlorid (11,5 g, 73,7 mmol) innerhalb von einer Stunde zugetropft. Nach 22 h Rühren bei Raumtemperatur wurde das Reaktionsgemisch zweimal mit 100 ml Wasser gewaschen. Die organische Phase wurde mit Magnesiumsulfat getrocknet, das Methylenchlorid abdestilliert und der verbleibende ölige Rückstand im Vakuum getrocknet. Es wurden 11,6 g der Zielverbindung erhalten, deren Konstitution mittels NMR-Spektroskopie gesichert wurde.

### Beispiel 4: Herstellung von n-Butyl-N-isoxazolo-sulfonamid

In 72 ml THF wurden 6,2 g 3-Aminoisoxazol (73,7 mmol) und 10,2 ml Triethylamin (7,4 g, 73,7 mmol) bei Raumtemperatur gelöst. Zu dieser Lösung wurden ebenfalls bei Raumtemperatur 9,6 ml n-Butansulfonsäurechlorid (11,5 g, 73,7 mmol) innerhalb von einer Stunde zugetropft. Nach 22 h Rühren bei Raumtemperatur wurde das Reaktionsgemisch mit 200 ml Methylenchlorid verdünnt, dann zweimal mit 200 ml 1 N NaOH ausgeschüttelt. Die wässrige Phase wurde vorsichtig mit konzentrierter HCl auf pH 1-2 gestellt, dann zweimal mit 100 ml Methylenchlorid extrahiert. Nach Trocknen mit Magnesiumsulfat wurde die organische Phase vom Lösungsmittel befreit und der ölige Rückstand im Vakuum getrocknet. Es wurden 7,8 g der Zielverbindung erhalten, deren Konstitution mittels NMR-Spektroskopie gesichert wurde.

### Beispiel 5: Herstellung von n-Butyl-N-2-thiazolyl-sulfonamid

In 100 ml THF wurden 7,4 g 2-Aminothiazol (73,7 mmol) und 10,2 ml Triethylamin (7,4 g, 73,7 mmol) bei Raumtemperatur gelöst. Zu dieser Lösung wurden ebenfalls bei Raumtemperatur 9,6 ml n-Butansulfonsäurechlorid (11,5 g, 73,7 mmol) innerhalb von einer Stunde zugetropft. Nach 21 h Rühren bei Raumtemperatur wurden noch einmal 4 ml n-Butansulfonsäurechlorid (4,8 g, 30,8 mmol) zugesetzt und weitere 20 h bei Raumtemperatur gerührt. Das Reaktionsgemisch wurde zweimal mit 100 ml 1 N NaOH gewaschen und dann mit Wasser neutral (pH 6-7) gewaschen. Die organische Phase wurde nach Trocknen mit Magnesiumsulfat vom Lösungsmittel befreit. Die erhaltenen 9,0 g Rohprodukt wurden aus 80 ml tert.-Butylmethylether umkristallisiert. Es wurden 3,2 g der Zielverbindung erhalten, deren Konstitution mittels NMR-Spektroskopie gesichert wurde.

### Beispiel 6: Herstellung von n-Butyl-N-Morpholino-sulfonamid

In 40 ml Methylenchlorid wurden 3,8 g N-Aminomorpholin (36,9 mmol) und 5,1 ml Triethylamin (3,7 g, 36,9 mmol) bei Raumtemperatur gelöst. Zu dieser Lösung wurden ebenfalls bei Raumtemperatur 4,8 ml n-Butansulfonsäurechlorid (5,8 g, 36,9 mmol) innerhalb von einer Stunde zugetropft. Nach 20 h Rühren bei Raumtemperatur wurde das Reaktionsgemisch zweimal mit 50 ml Wasser ausgeschüttelt, die organische Phase mit Magnesiumsulfat getrocknet und eingeengt.

Die erhaltenen 6,7 g des Rohprodukts wurden in 30 ml tert.-Butylmethylether umkristallisiert. Die Konstitution der Zielverbindung wurde mittels NMR-Spektroskopie gesichert.

### Beispiel 7: Herstellung von n-Butyl-N-pyrazino-sulfonamid

In 72 ml Methylenchlorid wurden 7,0 g Aminopyrazin (73,7 mmol) und 10,2 ml Triethylamin (7,4 g, 73,7 mmol) bei Raumtemperatur gelöst. Zu dieser Lösung wurden ebenfalls bei Raumtemperatur 9,6 ml n-Butansulfonsäurechlorid (11,5 g, 73,7 mmol) innerhalb von 1 h zugetropft. Nach 20 h Rühren bei Raumtemperatur wurde der Ansatz mit 100 ml Wasser versetzt, dann zweimal mit 200 ml Methylenchlorid extrahiert. Die organische Phase wurde einmal mit 100 ml Wasser gewaschen, dann mit Magnesiumsulfat getrocknet. Das Rohprodukt in 200 ml Methylenchlorid wurde mit 200 ml 1 N NaOH extrahiert. Die wässrige Phase wurde mit konzentrierter HCl auf pH 1-2 gestellt, dann mit 200 ml Methylenchlorid extrahiert. Die organische Phase wurde mit Magnesiumsulfat getrocknet und eingeengt. Es wurden 4,0 g der Zielverbindung erhalten, deren Konstitution mittels NMR-Spektroskopie gesichert wurde.

### Beispiel 8: Herstellung von n-Butyl-N-phenyl-sulfonamid

In 108 ml Methylenchlorid wurden 9,9 ml Anilin (10,1 g, 108,4 mmol) und 15 ml Triethylamin (11,0 g, 108,4 mmol) bei 50°C gelöst. Zu dieser Lösung wurden ebenfalls bei 50°C 14,1 ml n-Butansulfonsäurechlorid (17,0 g, 108,4 mmol) innerhalb von 1 h zugetropft. Nach 15 Minuten Rühren bei 50°C wurde das Reaktionsgemisch zweimal mit 100 ml Wasser extrahiert. Das erhaltene Rohprodukt in 150 ml Methylenchlorid gelöst wurde mit 150 ml 1 N NaOH extrahiert. Die wässrige Phase wurde mit konzentrierter HCl auf pH 1-2 gestellt und zweimal mit 100 ml Methylenchlorid extrahiert. Nach Trocknen mit Magnesiumsulfat und Abdestillieren des Methylenchlorides wurden 17,0 g der Zielverbindung erhalten, deren Konstitution mittels NMR-Spektroskopie gesichert wurde.

### Beispiel 9: Synthese des Tetrabutylammoniumsalzes des n-Butyl-N-n-propyl-sulfonamidanions

Eine Lösung von 6,7 g n-Butyl-N-n-Propylsulfonamid (37,3 mmol) aus Beispiel 1 in 7,5 ml Methanol wurde bei Raumtemperatur zu 7,1 ml einer 30%igen Na-Methylatlösung (37,3 mmol) zugetropft. Es wurde eine Stunde bei Raumtemperatur nachgerührt, danach wurden 16,9 g einer 61,4%igen Lösung von Tetrabutylammoniumchlorid (37,3 mmol) in Isopropanol zugetropft. Man rührte eine weitere Stunde bei Raumtemperatur nach und filtrierte dann das ausgefallene NaCl ab. Das Filtrat wurde im Vakuum vom Lösungsmittel befreit. Der Rückstand wurde im Vakuum getrocknet, um letzte Lösungsmittelreste zu entfernen. Man erhielt 14,1 g eines öligen Produktes. Die Konstitution der Zielverbindung wurde mittels NMR-Spektroskopie gesichert.

### Beispiel 10 -15

Durch zu Beispiel 9 analoger Vorgehensweise wurden die Tetrabutylammonium-Salze der Sulfonamide aus den Beispielen 2, 3 und 5 - 8 hergestellt und durch NMR-Spektroskopie charakterisiert.

### Beispiel 16: Synthese des Tetrabutylphosphoniumsalzes des n-Butyl-N-n-propyl-sulfonamidanions

Eine Lösung von 6,7 g n-Butyl-N-n-propylsulfonamid (37,3 mmol) aus Beispiel 1 in 7,5 ml Methanol wurde bei Raumtemperatur zu 7,1 ml einer 30%igen Na-Methylatlösung (37,3 mmol) zugetropft. Es wurde eine Stunde bei Raumtemperatur nachgerührt, danach wurden 15,4 g einer 71,4%igen Lösung von Tetrabutylphosphoniumchlorid (37,3 mmol) in Isopropanol zugetropft. Man rührte eine weitere Stunde bei Raumtemperatur nach und filtrierte dann das ausgefallene NaCl ab. Das Filtrat wurde im Vakuum vom Lösungsmittel befreit. Der Rückstand wurde im Vakuum getrocknet, um letzte Lösungsmittelreste zu entfernen. Man erhielt 16,6 g eines öligen Produktes. Die Konstitution der Zielverbindung wurde mittels NMR-Spektroskopie gesichert.

### Beispiel 17 - 22

Durch zu Beispiel 16 analoger Vorgehensweise wurden die Tetrabutylphosphonium-Salze der Sulfonamide aus den Beispielen 2, 3 und 5-8 hergestellt und durch NMR-Spektroskopie charakterisiert.

### Beispiel 23: Synthese des Tri-n-butyltetradecylphosphoniumsalzes des n-Butyl-N-phenylsulfonamidanions

Eine Lösung von 2,2 g n-Butyl-N-phenylsulfonamid (10,5 mmol) aus Beispiel 8 in 7 ml Methanol wurde bei Raumtemperatur zu 2 ml einer 30%igen Na-Methylatlösung (10,5 mmol) zugetropft. Es wurde eine Stunde bei Raumtemperatur nachgerührt, danach wurden 4,6 g Tri-n-butyltetradecylphosphoniumchlorid (10,5 mmol) zugetropft. Man rührte eine weitere Stunde bei Raumtemperatur nach und filtrierte dann das ausgefallene NaCl ab. Das Filtrat wurde im Vakuum vom Lösungsmittel befreit. Der Rückstand wurde im Vakuum getrocknet, um letzte Lösungsmittelreste zu entfernen. Man erhielt 5,2 g eines öligen Produktes. Die Konstitution der Zielverbindung wurde mittels NMR-Spektroskopie gesichert.

### Beispiel 24

Durch zu Beispiel 23 analoger Vorgehensweise wurde das Tri-n-butyltetradecylphosphonium-Salz des Sulfonamids aus Beispiel 7 hergestellt. Die Charakterisierung erfolgte über NMR-Spektroskopie.

### Beispiel 25: Synthese des Tri-n-hexyltetradecylphosphoniumsalzes des n-Butyl-N-phenylsulfonamidanions

Eine Lösung von 2,2 g n-Butyl-N-phenylsulfonamid (10,5 mmol) in 7 ml Methanol wurde bei Raumtemperatur zu 2 ml einer 30%igen Na-Methylatlösung (10,5 mmol) zugetropft. Es wurde eine Stunde bei Raumtemperatur nachgerührt, danach wurden 5,4 g Tri-n-hexyltetradecylphosphoniumchlorid (10,5 mmol) zugetropft. Man rührte eine weitere Stunde bei Raumtemperatur nach und filtrierte dann das ausgefallene NaCl ab. Das Filtrat wurde im Vakuum vom Lösungsmittel befreit. Der Rückstand wurde im Vakuum getrocknet, um letzte Lösungsmittelreste zu entfernen. Man erhielt 6,3 g eines öligen Produktes. Die Konstitution der Zielverbindung wurde durch NMR-Spektroskopie gesichert.

### Beispiel 26

Durch zu Beispiel 25 analoger Vorgehensweise wurde das Tri-n-hexyltetradecylphosphonium-Salz des Sulfonamids aus Beispiel 7 hergestellt. Die Konstitution der Zielverbindung wurde durch NMR-Spektroskopie gesichert.

### Beispiele 27 bis 29: Erfindungsgemäße Oligomerisierungsreaktionen

### Allgemeine Vorschrift

In ein Glasgefäß mit Septumverschluss wurden die in den Beispielen 27 bis 29 der Tabellen 1 bis 3 angegebenen Mengen an reinem Katalysator eingewogen. Dann wurde das Gefäß zweimal evakuiert und mit Argon gefüllt. Mit Hilfe einer Spritze wurden dann die ebenfalls in den Beispiele 27 - 29 der Tabellen 1-3 angegebenen Mengen Diisocyanat über das Septum zugegeben.

In den Fällen, bei denen der Katalysator als Lösung verwendet wurde (Beispiele 27 c, d, e, f; 28 c, d, h, i; 29 a, b, g, h), wurde das Reaktionsgefäß mit Septumverschluss zweimal evakuiert und mit Argon gefüllt. In das so vorbereitete Gefäß wurde mit Hilfe einer Spritze jeweils 5 ml Diisocyanat eingefüllt und danach unter Rühren die entsprechenden Mengen an Katalysator in dem angegebenen Lösungsmittel zugegeben.

Das erhaltene Reaktionsgemisch wurde anschließend in einem Ölbad oder in einem Rührheizblock (z.B. Variomag Reaktionsblock Typ 48.2/RM der Firma H&P Labortechnik GmbH, Oberschleißheim, Deutschland) unter den in den nachfolgenden Tabellen angegebenen Bedingungen umgesetzt.

Die anschließende Analytik erfolgte wie oben angegeben.

**Tabelle 1: Resultate der erfindungsgemäßen HDI Oligomerisierung**

| Bsp. | Kat. | Menge [mol-%] | Einsatzform | Zeit [h] | T. [°C] | Umsatz [%] | Typ 1 [mol-%] | Typ 2 [mol-%] |
|---|---|---|---|---|---|---|---|---|
| 27a | 15 | 0,025 | 100 %-ig | 0,33 | 40 | 50 | 0 | 100 |
| 27b | 22 | 0,025 | 100 %-ig | 0,25 | 40 | 37 | 0 | 100 |
| 27c | 14 | 0,75 | 1 M / i-PrOH | 1,5 | 40 | 63 | 0 | 100 |
| 27d | 21 | 0,75 | 1 M / i-PrOH . | 1 | 40 | 50 | 0 | 100 |
| 27e | 24 | 0,3 | 2 M / i-PrOH | 4 | 40 | 36 | 0 | 100 |
| 27f | 26 | 0,4 | 2,2 M / i-PrOH | 4 | 40 | 34 | 0 | 100 |

**Tabelle 2: Resultate der erfindungsgemäßen IPDI-Oligomerisierung**

| Bsp. | Kat. | Menge [mol-%] | Einsatzform | Zeit [h] | T [°C] | Umsatz [%] | Typ 1 [mol-%] | Typ 2 [mol-%] |
|---|---|---|---|---|---|---|---|---|
| 28a | 16 | 0,45 | 100 %-ig | 4 | 40 | 31 1 | 0 | 100 |
| 28b | 17 | 0,3 | 100 %-ig | 1 | 40 | 41 | 0 | 100 |
| 28c | 18 | 0,75 | 1 M /DMSO | 3 | 40 | 49 | 30 | 70 |
| 28d | 18 | 1,0 | 1 M / DMSO | 3 | 40 | 52 | 23 | 77 |
| 28e | 12 | 1,5 | 100 %-ig | 92 | 40 | 28 | 46 | 54 |
| 28f | 12 | 2,0 | 100 %-ig | 92 | 40 | 42 | 42 | 58 |
| 28g | 19 | 2,0 | 100 %-ig | 72 | 40 | 43 | 46 | 54 |
| 28h | 13 | 0,13 | 0,5 M / DMSO | 0,4 | 40 | 65 | 14 | 86 |
| 28i | 13 | 0,15 | 0,5 M / DMSO | 0,4 | 40 | 74 | 14 | 86 |
| 28j | 20 | 1,5 | 100 %-ig | 1 | 40 | 85 | 40 | 60 |
| 28k | 15 | 0,2 | 100 %-ig | 1 | 40 | 41 | 0 | 100 |
| 281 | 22 | 0,75 | 100 %-ig | 47 | 40 | 31 | 49 | 51 |
| 28m | 22 | 1,0 | 100 %-ig | 47 | 40 | 40 | 46 | 54 |
| 28n | 24 | 1,0 | 100 %-ig | 48 | 40 | 42 | 37 | 63 |

**Tabelle 3: Resultate der erfindungsgemäßen H₁₂MDI-Oligomerisierung**

| Bsp. | Kat. | Menge [mol-%] | Einsatzform | Zeit [h] | T [°C] | Umsatz [%] | Typ 1 [mol-%] | Typ 2 [mol-%] |
|---|---|---|---|---|---|---|---|---|
| 29a | 9 | 0,15 | 1 M / i-PrOH | 1,5 | 40 | 30 | 0 | 100 |
| 29b | 16 | 0,25 | 1 M / i-PrOH | 6 | 40 | 22 | 0 | 100 |
| 29c | 10 | 0,75 | 100 %-ig | 0,58 | 40 | 37 | 28 | 72 |
| 29d | 10 | 1,0 | 100 %-ig | 0,58 | 40 | 55 | 32 | 68 |
| 29e | 17 | 1,0 | 100 %-ig | 47 | 40 | 40 | 33 | 67 |
| 29f | 17 | 2,0 | 100 %-ig | 47 | 40 | 40 | 37 | 63 |
| 29g | 11 | 0,13 | 1 M / i-PrOH | 3 | 40 | 34 | 0 | 100 |
| 29h | 13 | 0,2 | 0,5 M / DMSO | 5 | 40 | 53 | 0 | 100 |
| 29i | 15 | 0,25 | 100 %-ig | 17 | 40 | 53 | 0 | 100 |

### Vergleichsbeispiele 1 bis 3

Die Umsetzungen von HDI, IPDI und H₁₂MDI wurden in Anlehnung an EP-A 0 010 589 mit Benzyltrimethylammoniumhydroxid als Lösung in Methanol (Triton^{®} B, Fa. Aldrich) analog der Vorgehensweise der erfindungsgemäßen Beispiele durchgeführt:

**Vergleichsbeispiel 1 a: Umsetzung von HDI**

| Bsp. | Katalysator | Menge [mol-%] | Zeit [h] | T [°C] | Umsatz [%] | Typ 1 [mol-%] | Typ 2 [mol-%] | Typ 3 [mol-%] |
|---|---|---|---|---|---|---|---|---|
| 1a | Triton^{®} B | 0,035 | 0,25 | 60 | 42,7 | 2,1 | 94,4 | 3,5 |

**Vergleichsbeispiel 2 a: Umsetzung von IPDI**

| Bsp. | Katalysator | Menge [mol%] | Zeit [h] | T [°C] | Umsatz [%] | Typ 1 [mol-%] | Typ 2 [mol-%] |
|---|---|---|---|---|---|---|---|
| 2a | Triton^{®} B | 0,18 | 1 | 40 | 48,6 | 0 | 100 |

**Vergleichsbeispiel 3 a: Umsetzung von H₁₂MDI**

| Bsp. | Katalysator | Menge [mol%] | Zeit [h] | T [°C] | Umsatz [%] | Typ 1 [mol-%] | Typ 2 [mol-%] |
|---|---|---|---|---|---|---|---|
| 3a | Triton^{®} B | 0,2 | 21,5 | 40 | 51,7 | 1,2 | 98,8 |

Wie man sehen kann, ist das salzartig aufgebaute Tetraalkylammoniumhydroxid sehr aktiv, liefert aber nur geringe Uretdionanteile im Produktgemisch. Im Fall der HDI-Umsetzung ist sogar eine deutliche Bildung von Iminooxadiazindion-Strukturen zu beobachten. Dagegen liefern die erfindungsgemäßen Katalysatoren, abhängig von ihrem Substitutionsmuster an Schwefel- und Stickstoff sehr variable Verhältnisse an Uretdion/Isocyanurat bei ebenfalls sehr hoher katalytischer Aktivität. Darüber hinaus ist bei Umsetzung des linearaliphatischen HDI eine beeindruckend hohe Selektivität bezüglich der Isocyanurat-Struktur zu beobachten, wobei gleichzeitig die Bildung des asymmetrischen Trimers (Typ 3) vollständig unterbleibt.

### Beispiel 30: Verfahrensbeispiel

### Trimerisierung von 4,4'-Diisocyanatodicyclohexylmethan

500 g (1,91 mol) 4,4'-Diisocyanatodicyclohexylmethan wurden 30 min im Vakuum (2 mbar) entgast, anschließend mit trockenem Stickstoff belüftet und auf 60°C erwärmt. Unter Rühren wurden nun 2,3 g (3,3 mmol) einer 60 %igen Katalysatorlösung des Katalysators des Beispiels 9, gelöst in 2-Ethyl-1,3-hexandiol, über einen Zeitraum von 1 Stunde so zugegeben, dass die Temperatur im Reaktionsgemisch 70°C nicht überstieg. Nach beendeter Katalysatorzugabe wurde die Trimerisierungsreaktion durch Zugabe von 0,7 g (3,3 mmol) Dibutylphosphat abgestoppt. Der NCO-Gehalt der Mischung betrug 25,8 %, entsprechend einem Oligomerisierungsgrad von 19,0 %. Die klare schwach gelbe Rohlösung wird nun mit 26,5 g eines Isocyanuratpolyisocyanats auf Basis von HDI, das nach Beispiel 12 der EP-A 330 966 erhalten wurde, versetzt und anschließend wie in Beispiel 2 beschrieben durch Dünnschichtdestillation von überschüssigem 4,4'-Diisocyanatodicyclohexylmethan befreit. Das anfallende Festharz wurde mit einem Gemisch aus 1-Methoxypropyl-2-acetat und Xylol (1 : 1) gelöst und auf einen Festkörpergehalt von 70 % eingestellt. Man erhielt eine helle klare Polyisocyanat-Lösung mit einem NCO-Gehalt von 10,4 %, einem Gehalt an monomerem 4,4'-Diisocyanatodicyclohexylmethan von 0,2 % und einer Viskosität (23°C) von 6.060 mPas.

## Patentansprüche

1. Verwendung von Sulfonamid-Salzen der Formel (I) 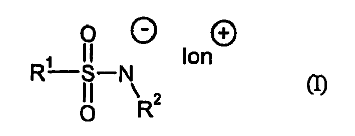 wobei
R¹, R² unabhängig voneinander gleiche oder verschiedene gegebenenfalls verzweigte, substituierte und/oder Heteroatom-enthaltende aliphatische, cycloaliphatische, aromatische oder araliphatische Reste sind und
Ion⁽⁺⁾ ein anorganisches oder organisches Kation ist,
zur Oligomeriserung von Isocyanaten.

2. Verwendung von Sulfonamid-Salzen der Formel (I) zur Oligomeriserung von Isocyanaten gemäß Anspruch 1, **dadurch gekennzeichnet, dass**
R¹ ein gegebenenfalls verzweigter aliphatischer oder cycloaliphatischer C₁-C₁₈ Rest ist, der gegebenenfalls bis zu 3 Heteroatome der Elemente Sauerstoff, Schwefel, Stickstoff und/oder gegebenenfalls Halogen-, Cyanid-, Nitro-, Alkyl-, Aryl-, Alkoxy-, Aryloxy- und/oder Dialkylamino-Substituenten aufweist,
R² ein Rest gemäß der Definition von R¹ ist oder Phenyl-, Pyrrolidin-, Piperidin-, Piperazin-, Morpholin-, 2-Pyrimidinyl-, 2-Thiazolyl-, 2-Benzthiazolyl-, 2-Pyrazyl-, 2-Pyridyl- oder 4-Pyridyl- ist und
Ion(+) Li⁺, Na⁺, K⁺ oder ein einwertiges Ammonium- oder Phosphoniumkation der allgemeinen Formel (II) 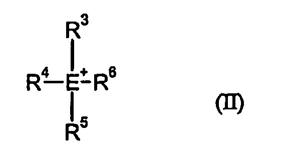
ist, in welcher
E Stickstoff oder Phosphor ist und
R³, R⁴, R⁵, R⁶ unabhängig voneinander gleiche oder verschiedene aliphatische, cycloaliphatische oder araliphatische gegebenenfalls Heteroatom-enthaltende C₁ - C₁₈ Reste sind.

3. Verfahren zur Oligomerisierung von Isocyanaten, bei dem
a) ein oder mehrere organische Verbindungen einer mittleren NCO-Funktionalität ≥ 1 in Anwesenheit
b) eines Katalysators enthaltend ein oder mehrere Sulfonamid-Salze gemäß Anspruch 1 oder 2 und
c) optional Lösungsmitteln
oligomerisiert werden.

4. Verfahren zur Oligomerisierung von Isocyanaten gemäß Anspruch 3, **dadurch gekennzeichnet, dass** die NCO-Oligomerisierung bei einer Temperatur von 20 - 100°C so lange durchgeführt wird, bis 10 - 60 mol-% aller NCO-Gruppen umgesetzt wurden und die Oligomerisierungsreaktion dann durch Zugabe eines Katalysatorgiftes abgebrochen und nicht umgesetztes monomeres Isocyanat durch Destillation abgetrennt wird.

## Claims

1. Use of sulphonamide salts of the formula (I) 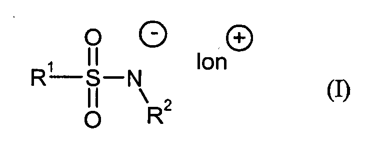 where
R¹, R² independently of one another are identical or different aliphatic, cycloaliphatic, aromatic or araliphatic radicals which are optionally branched, substituted and/or heteroatom-contained and
Ion⁽⁺⁾ is an organic or inorganic cation
for oligomerizing isocyanates.

2. Use of sulphonamide salts of the formula (I) for oligomerizing isocyanates according to Claim 1, **characterized in that**
R¹ is an optionally branched aliphatic or cycloaliphatic C₁-C₁₈ radical which optionally contains up to 3 heteroatoms of the elements oxygen, sulphur and nitrogen and/or optionally contains halogen, cyanide, nitro, alkyl, aryl, alkoxy, aryloxy and/or dialkylamino substituents,
R² is a radical as defined for R¹ or is phenyl, pyrrolidine, piperidine, piperazine, morpholine, 2-pyrimidinyl, 2-thiazolyl, 2-benzthiazolyl, 2-pyrazyl, 2-pyridyl or 4-pyridyl, and
Ion(+) is Li⁺, Na⁺ or K⁺ or a monovalent ammonium or phosphonium cation of the general formula (II) 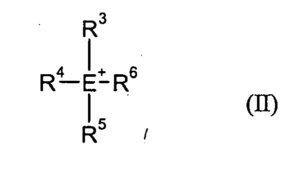
in which
E is nitrogen or phosphorus and
R³, R⁴, R⁵ and R⁶ independently of one another are identical or different aliphatic, cycloaliphatic or araliphatic, optionally heteroatom-contained C₁-C₁₈ radicals.

3. Process for oligomerizing isocyanates wherein
a) one or more organic compounds having an average NCO functionality ≥ 1 are oligomerized in the presence
b) of a catalyst comprising one or more sulphonamide salts according to Claim 1 or 2 and
c) optionally solvents.

4. Process for oligomerizing isocyanates according to Claim 3, **characterized in that** the NCO oligomerization is carried out at a temperature of 20 - 100°C until 10 - 60 mol% of all the NCO groups have undergone conversion and then the oligomerization reaction is terminated by addition of a catalyst poison and unreacted monomeric isocyanate is separated off by distillation.

## Revendications

1. Utilisation de sels de sulfonamides de formule (I) 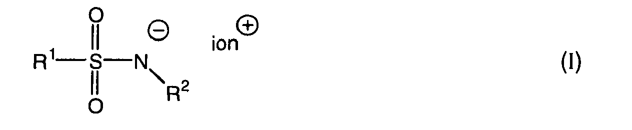 dans laquelle
R¹, R² indépendamment l'un de l'autre sont des radicaux aliphatiques, cycloaliphatiques, aromatiques ou araliphatiques, identiques ou différents, éventuellement ramifiés, substitués et/ou contenant des hétéroatomes et
ion⁽⁺⁾ est un cation inorganique ou organique,
pour l'oligomérisation d'isocyanates.

2. Utilisation de sels de sulfonamides de formule (I) pour l'oligomérisation d'isocyanates selon la revendication 1, **caractérisée en ce que**
R¹ est un radical aliphatique ou cycloaliphatique en C₁-C₁₈, éventuellement ramifié, qui contient éventuellement jusqu'à 3 hétéroatomes des éléments oxygène, soufre ou azote et/ou éventuellement présente des substituants halogène, cyanure, nitro, alkyle, aryle, alcoxy, aryloxy et/ou dialkylamino,
R² est un radical selon la définition de R¹ ou est un radical phényle, pyrrolidine, pipéridine, pipérazine, morpholine, 2-pyrimidinyle, 2-thiazolyle, 2-benzothiazolyle, 2-pyrazyle, 2-pyridyle ou 4-pyridyle et
ion⁽⁺⁾ est Li⁺, Na⁺, K⁺ ou un cation monovalent ammonium ou phosphonium de formule générale (II) 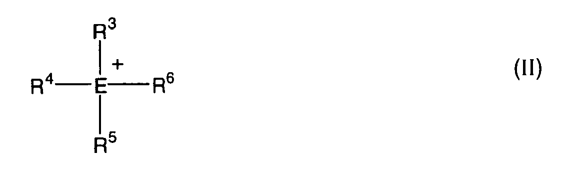
dans laquelle
E est l'azote ou le phosphore et
R³, R⁴, R⁵, R⁶ indépendamment l'un de l'autre sont des radicaux identiques ou différents en C₁-C₁₈, aliphatiques, cycloaliphatiques ou araliphatiques, contenant éventuellement un hétéroatome.

3. Procédé d'oligomérisation d'isocyanates selon lequel on oligomérise
d) un ou plusieurs composés organiques d'une fonctionnalité NCO moyenne ≥ 1 en présence
e) d'un catalyseur contenant un ou plusieurs sels de sulfonamide selon la revendication 1 ou 2 et
f) éventuellement des solvants.

4. Procédé d'oligomérisation d'isocyanates selon la revendication 3, daractérisé en ce qu'on effectue l'oligomérisation de NCO à une température de 20 à 100 °C jusqu'à ce que 10 à 60 % en moles de tous les groupes NCO soient convertis et on arrête la réaction d'oligomérisation par addition d'un poison de catalyseur et on sépare par distillation l'isocyanate monomère n'ayant pas réagi.
